# EUROPEAN PATENT SPECIFICATION

(11) **EP 1 036 847 B1**
(45) Date of publication and mention of the grant of the patent: **27.10.2004**
(21) Application number: 00300791.1
(22) Date of filing: 01.02.2000
(51) Int. Cl.: C12Q 1/70, C12Q 1/68

(54) **Oligonucleotide primers for efficient reverse transcription of Hepatitis C Virus (HCV) RNA and methods of use thereof**
Oligonukleotid Primer zur effizienten reversen Transkription von Hepatitis C Virus (HCV) RNS und Verfahren zu deren Verwendung
Amorces oligonucléotidiques pour une transcription inverse efficace de l'ARN du virus de l'Hépatite C et ses procédés d'utilisation

(30) Priority: 03.02.1999 US 118520 P
(43) Date of publication of application: 20.09.2000
(73) Proprietor: Ortho-Clinical Diagnostics, Inc., Rochester, NY 14626-5101 (US)
(72) Inventor: Linnen, Jeffrey M., San Diego, California 92129 (US); Gorman, Kevin M., Rochester, New York 14616 (US)
(74) Representative: Fisher, Adrian John

(56) References cited:
- EP-A- 0 633 320
- EP-A- 0 776 981
- WO-A-97/08310
- WO-A-99/04008
- US-A- 5 837 463
- KHORSI H ET AL: "Amplification and detection of the terminal 3' non-coding region of hapatitis C virus isolates" RESEARCH IN VIROLOGY, vol. 149, 1998, pages 115-2516, XP000929301
- UMLAUFT F ET AL: "Hepatitis C Virus detection by single-round PCR specific for the terminal 3' noncoding region" JOURNAL OF CLINICAL MICROBIOLOGY, vol. 34, October 1996 (1996-10), page 2552-58 XP000929388
- BUKH J ET AL: "IMPORTANCE OF PRIMER SELECTION FOR THE DETECTION OF HEPATITIS C VIRUS RNA WITH THE POLYMERASE CHAIN REACTION ASSAY" PROCEEDINGS OF THE NATIONAL ACADEMY OF SCIENCES OF USA,US,NATIONAL ACADEMY OF SCIENCE. WASHINGTON, vol. 89, 1992, pages 187-191, XP002059829 ISSN: 0027-8424

## Description

### Field of the Invention

The present invention pertains to improved methods for detecting nucleic acid sequences in biological samples, particularly sequences derived from infectious microorganisms.

### Background of the Invention

Hepatitis C Virus (HCV) is a parenterally transmitted virus responsible for the majority of cases of post-transfusion hepatitis and a substantial portion of sporadic (or community acquired) hepatitis cases worldwide. It is estimated that more than 1% of the world's population is infected with HCV. HCV infection is associated with acute hepatitis (which is often mild), chronic hepatitis (of varying severity), cirrhosis, and subsequent hepatocellular carcinoma.

HCV is currently classified as a separate genus, Hepacivirus, in the family Flaviviridae. Its genome consists of a positive-stranded RNA molecule of about 9,500 nucleotides with a single, large open reading frame (ORF) which encodes a polyprotein precursor of about 3,000 amino acids. The large ORF is preceded by a 5' non-coding (NC) region of about 340 nucleotides, which is the most highly conserved region of the genome. The 5' region of the ORF encodes (in a 5'-to-3' direction) a capsid protein, two envelope glycoproteins (E1 and E2), and a small protein of unknown function (P7). The 3' portion of the ORF encodes nonstructural proteins which include a protease, protease/helicase bi-functional protein, RNA polymerase, and regulatory peptides.

Analysis of HCV coding sequences from around the world has revealed considerable sequence variation among individual viral isolates. Furthermore, analyses of HCV sequences from individual patients have shown that the virus circulates as so-called "quasi-species," which contain related but not identical sequences. The variation that exists among isolates and within individual patients is believed to be the result of the low fidelity of the virally-encoded RNA-dependent RNA polymerase. The degree of genetic variability of HCV has important implications for prevention, diagnosis, and control of infection.

Serodiagnosis of HCV infection is typically determined by commercially available enzyme immuno-assays (EIA) which detect antibodies that bind recombinant HCV proteins peptides. Positive EIA results can be confirmed by a recombinant immunoblot assay (RIBA), but neither EIA nor RIBA assays distinguish past from present infections. Because of the typically low titer of circulating virus, a direct assay for viral proteins has not been successfully developed. Furthermore, antibody-based assays fail to detect HCV infection for usually 2 to 3 months after exposure

Thus, there is a need in the art for improved assays for HCV that are sensitive enough to detect HCV viremia within a few days after initial exposure of a patient to HCV.

Research in Virology, vol. 149, 1998, pages 115-121; Journal of Clinical Microbiology, vol. 34, october 1996, pages 2552-2558; and WO 99/04008 relate to the amplification of HCV using an oligonucleotide sequence located in the 3' untranslated region of the genome.

### Summary of the Invention

Thus, in one aspect, the present invention is directed to a method for reverse transcribing Hepatitis C Virus (HCV) RNA in a biological sample. The method comprises:
(a) contacting RNA derived from the sample with an oligonucleotide under conditions in which the oligonucleotide primes synthesis of DNA complementary to at least a portion of the RNA;
   where the oligonucleotide is selected from the group consisting of:
   (i) 
5'-
AGGCCAGTATCAGCACTCTCTGCAGTC-3' (57R27) <SEQ ID NO.: 1>,
   (ii) 
5'-GTATCAGCACTC-3' (66RT12) <SEQ ID
NO.: 2>,
   (iii) 
5'-AGTATCAGCACTC-3' (66RT13) <SEQ
ID NO.: 3>,
   (iv) 
5'-CAGTATCAGCACTC-3' (66RT14) <SEQ
ID NO.: 4>,
   (v) 
5'-CCAGTATCAGCACTC-3' (66RT15)
<SEQ ID NO.: 5>,
   (vi) 
5'-GCCAGTATCAGCACTC-3' (66RT16)
<SEQ ID NO.: 6.>,
 and
   (vii) any combination of any of the foregoing.

In one embodiment, the method further comprises (b) recovering said cDNA.

In another aspect, the present invention is directed to a method for detecting the presence of Hepatitis C Virus (HCV) RNA in a biological sample. The method comprises:
(a) performing a reverse transcription reaction using as a template, RNA derived from the sample and using as a primer an oligonucleotide complementary to a sequence contained within the RNA to produce HCV-specific reverse transcription products, where the primer is selected from the group consisting of:
   (i) 
5'-
AGGCCAGTATCAGCACTCTCTGCAGTC-3' (57R27) <SEQ ID NO.: 1>,
   (ii) 
5'-GTATCAGCACTC-3' (66RT12) <SEQ ID
NO. 2>,
   (iii) 
5'-AGTATCAGCACTC-3'   (66RT13)   <SEQ
ID NO.: 3>,
   (iv) 
5'-CAGTATCAGCACTC-3' (66RT14) <SEQ
ID NO.: 4>,
   (v) 
5'-CCAGTATCAGCACTC-3' (66RT15)
<SEQ ID NO.: 5>,
   (vi) 
5'-GCCAGTATCAGCACTC-3' (66RT16)
<SEQ ID NO.: 6>,
 and
   (vii) any combination of any of the foregoing.
(b) amplifying products of the reverse transcription reaction to produce amplification products; and
(c) detecting the amplification products;
where detection of the amplification products indicates the presence of HCV RNA in the sample.

Amplification may be carried out by any method, preferably polymerase chain reaction (PCR). The use of HCV-specific reverse transcription primers according to the invention provides a sensitive method for detecting HCV in a sample, preferably plasma.

In yet another aspect, the present invention is directed to a kit for detection of HCV in a biological sample. The kit comprises a reverse transcription primer selected from the group consisting of:
(i) 
5'-AGGCCAGTATCAGCACTCTCTGCAGTC-3'
(57R27) <SEQ ID NO.: 1>,
(ii) 
5'-GTATCAGCACTC-3' (66RT12) <SEQ ID NO.:
2>,
(iii) 
5'-AGTATCAGCACTC-3' (66RT13) <SEQ ID NO.:
3>,
(iv) 
5'-CAGTATCAGCACTC-3' (66RT14) <SEQ ID NO.:
4>,
(v) 
5'-CCAGTATCAGCACTC-3' (66RT15) <SEQ ID
NO.: 5>,
(vi) 
5'-GCCAGTATCAGCACTC-3' (66RT16) <SEQ ID
NO.: 6>,
 and
(vii) any combination of any of the foregoing.
The kits may additionally comprise reagents and instructions for reverse transcription, amplification, and product detection.

### Detailed Description of the Invention

The present inventors have discovered that detection of Hepatitis C Virus (HCV) RNA in biological samples is more efficient when oligonucleotides having sequences complementary to certain sequences present in HCV RNA are used as primers for reverse transcription reactions. Preferably, the sequences of the primers correspond to sequences near the 3' non-coding region of HCV RNA.

The 3' non-coding (NC) region of HCV is only 98 nucleotides long, which is too short to support the use of random primers to prime the reverse transcription process in this region. The present invention provides novel, specific oligonucleotide primers that allow reverse transcription (and hence amplification and detection) of sequences derived from the 3' non-coding region of the HCV genome.

Many techniques in molecular biology, microbiology, recombinant DNA, and protein biochemistry are used in practicing the present invention, such as those explained in, for example, Current Protocols in Molecular Biology, Volumes I, II, and III, 1997 (F.M.. Ausubel ed.); Sambrook et al., 1989, Molecular Cloning: A Laboratory Manual, Second Edition, Cold Spring Harbor Laboratory Press, Cold Spring Harbor, New York; DNA Cloning: A Practical Approach, Volumes I and II, 1985 (D.N. Glover ed.); Oligonucleotide Synthesis, 1984, (M.L. Gait ed.); Transcription and Translation, 1984 (Hames and Higgins eds.); A Practical Guide to Molecular Cloning; the series, Methods in Enzymology (Academic Press, Inc.); and Protein Purification: Principles and Practice, Second Edition (Springer-Verlag, N.Y.).

"Nucleic acid" or "polynucleotide" as used herein refers to purine- and pyrimidine-containing polymers of any length, either polyribonucleotides or polydeoxyribonucleotides or mixed polyribo-polydeoxyribo nucleotides. This includes single- and double-stranded molecules, such as, for example, DNA-DNA, DNA-RNA and RNA-RNA hybrids, as well as "protein nucleic acids" (PNA) formed by conjugating bases to an amino acid backbone. This also includes nucleic acids containing modified bases.

A "complement" of a nucleic acid sequence as used herein refers to the antisense sequence that participates in Watson-Crick base-pairing with the original sequence.

A "primer" as used herein is an oligonucleotide between about 10 and about 50 nucleotides in length, preferably between about 12 and about 25 nucleotides in length and most preferably between about 12 and about 18 nucleotides in length, that forms a duplex with a single-stranded nucleic acid sequence of interest and allows polymerization of a complementary strand using, e.g., reverse transcriptase or DNA polymerase.

An "isolated" nucleic acid or polypeptide as used herein refers to a component that is removed from its original environment (for example, its natural environment if it is naturally occurring or a reaction mixture if it is synthetic). An isolated nucleic acid or polypeptide typically contains less than about 50%, preferably less than about 75%, and most preferably less than about 90%, of the components with which it was originally associated.

A nucleic acid sequence that is "derived from" a designated sequence refers to a sequence that corresponds to a region of the designated sequence. This encompasses sequences that are homologous or complementary to the sequence.

An internal positive control (IPC) target nucleic acid refers to a synthetic nucleic acid sequence cloned into a plasmid vector which is subsequently linearized, typically by the action of a restriction endonuclease. An IPC will typically have multiple primer binding sequences surrounding a generic probe-binding region, and acts as a generic control against false negative results in nucleic acid amplification reactions.

The sequence of a preferred internal positive control target DNA is:

5'-
CGCCAGCGTGGACCATCAAGTAGTAATGAACGCACGGACGAGGACATCA
TAGAGATTACACCTTTATCCACAGTTCTCGGTCTAACGCAGCAGTCAGTG
TATCAGCACCAGCATCCGTAGTGAGTCTTCAGTGTCTGCTCCAGGATCGT
G-3' <SEQ ID NO.: 7>.

As used herein, conditions appropriate for reverse transcription, i.e., conditions in which an oligonucleotide will prime cDNA synthesis, encompass incubation of RNA and primer oligonucleotides with a reverse transcriptase enzyme and nucleotides at a temperature and for a time that results in synthesis of cDNA (see, e.g., Sambrook et al.)

Nucleic acids comprising any of the sequences disclosed herein or subsequences thereof can be prepared by conventional methods. For example, DNA can be chemically synthesized using, e.g., the phosphoramidite solid support method of Matteucci et al., 1981, J. Am. Chem. Soc. 103:3185, the method of Yoo et al., 1989, J. Biol. Chem. 764:17078, or other well known methods. The nucleic acids may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) or charged linkages (e.g., phosphorothioates, phosphorodithioates, etc.). Nucleic acids may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. PNAs are also encompassed by the term "nucleic acid". The nucleic acid may be derivatized by formation of a methyl or ethyl phosphotriester or an alkyl phosphoramidate linkage. Furthermore, the nucleic acid sequences of the present invention may also be modified with a label capable of providing a detectable signal, either directly or indirectly. Exemplary labels include radioisotopes, fluorescent molecules, biotin, and the like.

Amplification as used herein refers to an iterative process by which a nucleic acid is copied. Suitable methods for amplification include without limitation polymerase chain reaction, ligase chain reaction, strand displacement reaction, nucleic acid single base amplification, and transcription mediated amplification.

Hepatitis C Virus (HCV) as used herein refers to viruses in the genus Hepacivirus. Isolates of HCV that may be detected by the present invention include, but are not limited to, HCV serotypes 1-6.

The present invention provides methods for reverse transcribing HCV RNA in biological samples. Efficient reverse transcription of HCV RNA allows for sensitive detection of HCV in biological samples. Reverse transcription of HCV RNA is carried out by contacting DNA derived from the sample with an oligonucleotide of the invention under conditions in which the oligonucleotide primes synthesis of DNA complementary to at least a portion of the RNA.

Detection of HCV RNA in a biological sample is performed by performing a reverse transcription reaction using as a template RNA contained within the sample or RNA derived from the sample, using as a reverse transcription primer an oligonucleotide of the invention which is complementary to a sequence contained within HCV RNA, to produce HCV-specific reverse-transcription products, followed by amplification of the reverse-transcription products to produce HCV-specific amplification products, followed by detection of HCV-specific amplification products. Detection of HCV-specific amplification products indicates the presence of HCV RNA in the sample.

According to the invention, a biological sample is obtained from a patient by any conventional means. Suitable biological samples include, without limitation, blood, serum, plasma, saliva, urine, breast milk, and cerebrospinal fluid. Preferably, plasma is used as the source of HCV RNA.

The biological sample is treated in any manner that provides access of the reverse transcription reagents to RNA, specifically HCV RNA, contained within the sample. RNA "derived from" a biological sample is any RNA which was originally present in the sample and to which access has been gained by treating the sample. Preferably, RNA is extracted from the sample using any method well known in the art, such as, e.g., methods employing guanidinium thiocyanate, or using commercially available reagents and methods such as, e.g., PureScript® from Gentra Systems, Inc. (Minneapolis MN). Any extraction procedure may be used that results in separation from the RNA of RNases, other proteins, and/or any other components that might interfere with reverse transcription.

The sample is then subjected to reverse transcription using primers derived from the sequence of HCV. Preferably, the primers correspond to regions of HCV RNA that are downstream, i.e., 3', to regions whose detection is desired. These regions may include, e.g., the regions encoding the viral capsid protein, E1 and E2 proteins, P7 protein, protease, protease/helicase, RNA polymerase, and regulatory proteins, as well as 5'- and 3'-noncoding regions. Preferably, the primers correspond to sequences in the 3' non-coding region, which includes a 98 bp nonhomopolymeric sequence at the extreme 3' terminus (Tanaka et al., J. Virol. 70: 3307, 1996; Kolykhalov et al., J. Virol. 70: 3363, 1996). The primer sequences may be used to specifically identify particular isolates or serotypes of HCV (e.g., HCV 1-6). A primer may identify a particular isolate or serotype by hybridizing to RNA derived from that isolate under conditions in which it does not hybridize to RNA from a different isolate, i.e., the primer itself may comprise a sequence that differs between isolates. Alternatively, the primer sequence may be used to prime synthesis of a fragment of HCV RNA that differs between isolates, i.e., the sequence that differs between the isolates may be downstream of the primer sequence.

Reverse transcription primers useful in practicing the present invention are selected based on theoretical considerations of sequence conservation, intra- and inter-molecular interactions, and the predicted secondary structures of the amplicon and surrounding sequence. Furthermore, the primers and assay system are designed to allow the co-amplification (and co-detection) of multiple regions of the HCV genome, multiple viral species, and an internal positive control (IPC) RNA (or DNA).

Non-limiting examples of HCV reverse transcription primers according to the invention include:
5'-AGGCCAGTATCAGCACTCTCTGCAGTC-3' <SEQ ID NO.: 1> (designated 57R27, corresponding to nucleotides 57-83 relative to the 3'-noncoding region of HCV); 5'-TCAGCACTCTCT-3' <SEQ ID NO.: 8> (designated 63RT12, corresponding to nucleotides 63-74); 5'-GTATCAGCACTC-3' <SEQ ID NO.: 2> (designated 66RT12, corresponding to nucleotides 66-77); and extended versions of 66RT12, i.e., 5'-AGTATCAGCACTC-3' <SEQ ID NO. 3> (66RT13); 5'-CAGTATCAGCACTC-3' <SEQ ID NO.: 4> (66RT14); 5'-CCAGTATCAGCACTC-3' <SEQ ID NO.: 5> (66RT15); and 5'-GCCAGTATCAGCACTC-3' <SEQ ID NO.: 6> (66RT16).

Reverse transcription is performed using one or more of the above primers. Random primers, such as, e.g., random hexamer reverse transcription primers (N6, Pharmacia Biotech, Piscataway, NJ) may also be added. Reverse transcription is carried out using conventional procedures, such as are described in Current Protocols in Molecular Biology, Volumes I, II, and III, 1997 (F.M.. Ausubel ed.); in U.S. Patent 5,322,770; in Young, et al., J. Clin. Microbiol. 31(4):882 (1993); Myers et al., Biochemistry 30(3):7661 (1991).

Following the reverse transcription reaction, the products are recovered, and may be amplified. Any method for amplification may be used, including, without limitation, the polymerase chain reaction (PCR), the ligase chain reaction, the strand displacement reaction, nucleic acid single base substitution, or transcription mediated amplification. Preferably, PCR is used. Typically, a reaction mixture containing all of the necessary components for PCR (including HCV-specific amplification primers) is added directly to the reverse transcription reaction mixture. Amplification is then carried out using conditions specified by the primer pairs that are used.

Following amplification, the amplification products may be detected using any method known in the art, including, without limitation, gel electrophoresis in agarose or acrylamide; capture of the amplification products on a solid support followed by colorimetric detection (see, e.g., Example 1 below), ECi detection; chemiluminescent detection, radioisotopic detection, and fluorescent detection. Reagents for such detection methods are commercially available from., e.g, Molecular Probes, Eugene, Oregon and Ortho Clinical Diagnostics, Rochester, NY.

The detection of HCV-specific amplification products indicates the presence of HCV RNA in the sample. When gel electrophoresis is used, HCV-specific amplification products are confirmed by their size, as predicted by the location in HCV RNA of the sequences corresponding to the amplification primers used in the reaction.

Kits for detection of HCV in a biological sample may be prepared which contain a reverse transcription primer selected from:
(i) 
5'-AGGCCAGTATCAGCACTCTCTGCAGTC-3'
(57R27) <SEQ ID NO.: 1>,
(ii) 
5'-GTATCAGCACTC-3' (66RT12) <SEQ ID NO.:
2>,
(iii) 
5'-AGTATCAGCACTC-3' (66RT13) <SEQ ID NO.:
3>,
(iv) 
5'-CAGTATCAGCACTC-3' (66RT14) <SEQ ID NO.:
4>,
(v) 
5'-CCAGTATCAGCACTC-3' (66RT15) <SEQ ID
NO.: 5>,
(vi) 
5'-GCCAGTATCAGCACTC-3' (66RT16) <SEQ ID
NO.: 6>,
 and
(vii) any combination of any of the foregoing.
The kits may additionally comprise reagents and instructions for reverse transcription, amplification, and product detection. For example, the kits can contain reverse transcriptase, deoxynucleotides, thermostable polymerases suitable for DNA amplification reactions, and reagents for labeling and detection of nucleic acids.

The methods and compositions of the present invention find use in the diagnosis of HCV infection in patients; in testing the efficacy of anti-HCV therapeutic regimens; and in screening the blood supply for HCV-infected samples.

### Description of the Preferred Embodiments

The following examples illustrate the present invention without limitation.

### Methods:

1. Sample preparation:
RNA was prepared from plasma samples using PureScript® RNA isolation reagents (Gentra Systems, Minneapolis MN). Modifications to the manufacturer's protocol for body fluids included use of 40 µg glycogen, rather than 20 µg, as a carrier to aid in the precipitation of viral RNA. Additionally, in most cases, after isopropyl alcohol precipitation of the RNA and washing the RNA pellet with ethanol, the RNA pellet was resuspended in the RT buffer mix, rather than in the RNA hydration solution provided by the manufacturer.

2. Reverse Transcription:
The synthesis of cDNA from RNA was catalyzed by the addition of 100 U recombinant Moloney Murine Leukemia Virus (M-MLV) reverse transcriptase (RT) (Gibco BRL, Gaithersburg, MD) in a 50 µl solution of 50 mM Tris-HCl (pH 8.3), 75 mM KCl, 3 mM MgCl2, 10 mM DTT, 0.4 mM of each dNTP (Pharmacia Biotech, Piscataway, NJ ), 4 µM primers, and 20 units RNasin (Promega, Madison, Wisconsin) in diethylpyrocarbonate (DEPC)-treated water. After incubation at 42°C for 30 min, the RT reaction was held at 100°C for 5 min to destroy RT activity. Each reaction was chilled for 1 min followed by microcentrifugation at 16000 x g for 4 seconds.

3. PCR amplification:
PCR was carried out IN A PE9600 thermocycler (Perkin-Elmer) in a 100 µl solution of 25 mM Tris-HCl, 3 mM MgCl2, 0.725 mM EDTA, 54 mM KCI, 3.72 mM NaCl, 40 µM DTT, 108 µg/ml gelatin (type IV), 9.5% glycerol, 0.02% Tween 20, 0.02% NP40, calf thymus DNA (2 µg), 1.2 mM of each dNTP, 0.4 µM each primer, 10 copies linearized internal positive control (IPC) plasmid DNA and 16 U of Taq polymerase. Monoclonal antibodies to Taq, TP1-12 and TP4-9, the preparation of which are disclosed in U.S. Patent 5,338,671, were added to the reaction at a 50:1 and 5:1 molar ratio, respectively, to provide a 55:1 molar ratio of antibody to Taq polymerase. After initial denaturation at 96°C for 3 min, 40 cycles of amplification were performed at 96°C for 5 sec and 68°C for 40 sec. At the conclusion of cycling, a post-heat step was performed for 5 min at 103°C to inactivate Taq polymerase. The primers used are shown in Table 3 below.

4. Detection of PCR products:
PCR products were biotinylated by use of 5'-biotin-labeled primers (sense strand) during amplification. Product was captured by hybridization to oligonucleotide probes covalently attached to latex particles, which were deposited on the surface of a flow through membrane (SureCell® tests, Ortho Clinical Diagnostics, Rochester, NY). The probes were: 5'-GCGGCTCACGGACCTTTCACAGCTA-3 <SEQ ID NO.: 9> and 5'-ATGCGGCTCACGGACCTTTCACAGC-3' <SEQ ID NO.: 10>. The probe/product complex was reacted with streptavidin (SA)-horseradish peroxidase (HRP) conjugate, which catalyzes the oxidative conversion of a dye precursor to a dye (blue color). The blue color intensity was scored visually (0-10) by comparing color intensity to color standards. All visual color scores > 3 were considered to be positive results.

5. Roche Amplicor Assay: The Roche Amplicor assay was performed according to the manufacturer's instructions (Roche Diagnostics, Kaiseraugst, Switzerland).

### Example 1: Design of HCV-Specific Reverse Transcription Primers Derived From the 3' Non-Coding Region

The following experiment was performed to test the efficiency of reverse transcription of HCV RNA derived from human plasma samples using HCV-specific 3' noncoding region primers.

An oligonucleotide primer having the sequence 5'-AGGCCAGTATCAGCACTCTCTGCAGTC-3' <SEQ ID NO:1:> (designated 57R27) was used to prime reverse transcription using HCV RNA as a template. Amplification was performed using as a forward primer 5'-GGTGGCTCCATCTTAGCCCTAGTCACG-3' <SEQ ID NO.: 11> (designated 1F27) and as a reverse primer 57R27.

The reaction yielded an 83 nt product, which was of the predicted size. However, the use of the 57R27 in a multiplex reverse transcription/amplification reaction, i.e., a reaction which also included an internal positive control (IPC), resulted in two non-specific (i.e., non-HCV) products of 100 and 70 nt.

### Example 2: Optimization of a 3' Non-coding Region HCV Reverse Transcription Primer for Use in Multiplex Reactions

The following experiment was performed to test the use of primers derived from 57R27 in multiplex reverse transcription/amplification assays.

As described above in Example 1, when multiplexed with IPC, the 57R27 reverse primer formed two "side product" bands that included an intense 70 bp and a less intense 100 bp gel band when stained with ethidium bromide. These side product bands were preferentially generated and as a result the HCV product band was usually absent. Closer analysis revealed that there was a strong interaction between 57R27 and the IPC reverse primers.

To overcome this problem, smaller reverse transcription (RT) primers were designed to eliminate the direct competition of RT primers with PCR primers during the PCR process. These new RT primers ranged from 8 to 16 bases in length, and their 3' ends were designed to be downstream from the 3' end of 57R27.

They were tested as follows: After RNA extraction and purification, reverse transcription was performed using the primers, and PCR was performed using the same primers as forward primers and the 27-mer reverse primer.

This new design had four distinct advantages.
1) The Tm of these primers was lower than the Tm of the 27-mer, therefore they were less likely to anneal and extend under the PCR conditions.
2) The 3' end of the RT primers was downstream from the 3' end of the 27-mer, therefore eliminating the possibility of cross reacting with the exact same target DNA as the 27-mer, and thus eliminating primer-dimers.
3) The positioning within the genome was critical to amplifying all genotypes and reverse primers were designed to correspond with conserved sequences within the HCV genome.
4) Multiplexed PCR was possible after reverse transcribing with short RT primers.

### A. Characteristics of 12-mer Primers

Four short RT primers (12-mers) were initially designed and were tested in pair-wise combinations. The goal was to determine (i) if there was any difference in resulting PCR band intensities between combined or single RT primers used in the reverse transcription process and (ii) if these short RT primers could be used in combination with each other. If short RT primers are used in combination it may be a benefit in avoiding mismatches at the 3' end.

The results indicate that there is no detrimental effect in using short RT primers in combination. Where it might be perceived that primers are being used in two fold concentration (primer being used with itself), the primer is actually being used at the standard concentration without being combined with any other primer.

Pair-wise combinations of 12-mer RT primers were used during reverse transcription as shown in the Table 1. PCR was performed using the 3' NC region primers 3x1F27 and 3x57R27 forward and reverse primers, respectively (see, Example 1 above). The HCV target was used at 50 copies per reaction. Standard RT-PCR conditions were used.

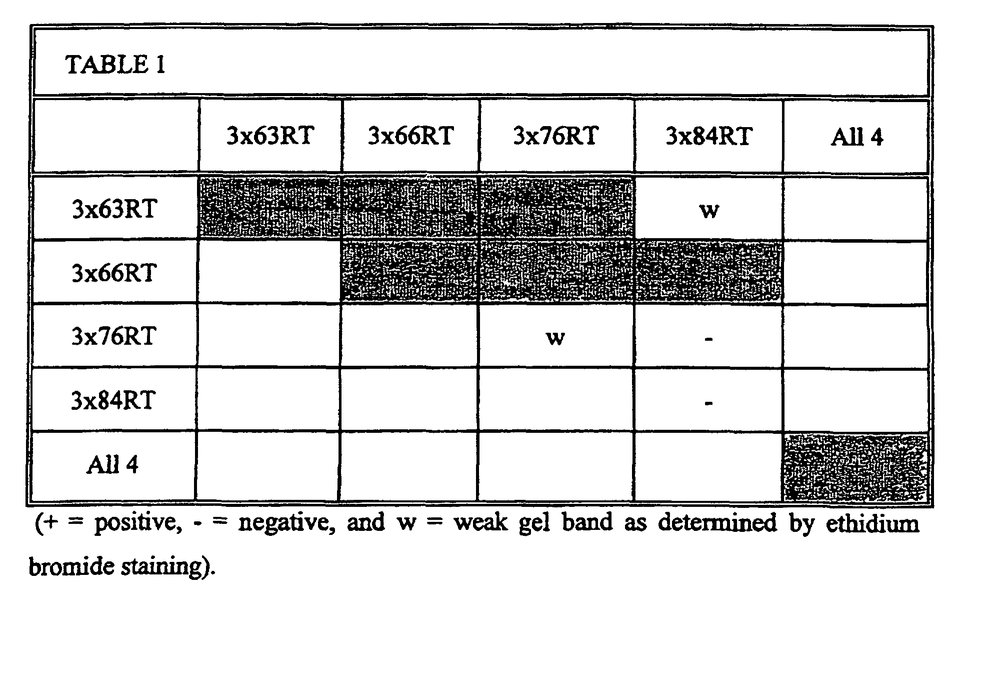

3x84RT failed to reverse transcribe when used either alone, or in combination with 3x76RT. It also seemed to have a detrimental effect on 3x63RT. Both 3x66RT and 3x63RT reverse transcribed well and the resulting (ethidium bromide-stained) PCR gel bands were strong regardless of which primer combination was used. There did not seem to be any advantage to combining 12-mer RT primers.

### B. Primer Concentration Range:

Both 3x63RT and 3x66RT were selected for further testing. The standard concentration for the 27-mer (3x57R27) had been at 1 µM for use in the reverse transcription process. The 12-mers, due to their shorter length, were potentially less likely to anneal and extend as efficiently as the 27-mer, and were thought to be needed at a greater concentration for reverse transcription. When used individually at 1, 2, 3 or 4 (M concentration, the RT-PCR results for 3x63RT, 3x66RT and the 27-mer were equivalent, regardless of the concentration. Standard practice was to use a 12-mer in the RT reaction, and to add the 27-mer (and 3x 1F27) as reverse and forward primers, respectively, PCR.

### C. Effect of Sample Treatment:

The 12-mer (3x66RT) and the 27-mer (3x57R27) reverse transcriptions were compared. Again, when the 12-mer was used as the reverse transcription primer, the 27-mer and forward primer (3x1F27) were used during the corresponding PCR. Samples were placed under varying conditions, and the resulting ethidium bromide-stained PCR gel bands were compared to determine if any condition had an effect on the gel band quality or intensity.

During the RNA preparation step, ice-cold isopropanol (IPA) was added to each sample tube and the tubes were rocked 2 min at room temp. RNA was allowed to further precipitate at -80°C (in IPA), or was immediately prepared at room temperature. Prior to performing the reverse transcription, RNA was resuspended in a complete RT mix and either placed on ice or left at room temperature or 10 min.

Reverse transcription was performed at either 37°C or 42°C. Prior to amplification, the cDNA either sat at room temperature in complete PCR mix for 4.5 hours, or was added immediately prior to performing the amplification reaction.

Results: The 12-mer RT primer (3x66RT) performed equally well in any of the aforementioned conditions. The 12-mer and 27-mer RT primers seemed to be equivalent in terms of band intensities, although the 12-mer was superior overall as the resulting PCR band was a single, clean band, with no side products.

### Example 3: Use of 12-mer Reverse Transcription Primers for Detection of HCV RNA at 50 Copies/Reaction

The following experiment was performed to test the ability of the 12-mer primers described above to detect HCV in patient samples containing HCV of different serotypes.

Genotyped patient plasma was quantified by the Roche Monitor® assay and serially diluted to 50 copies per reaction. RNA was extracted using a modified Purescript® method, and reverse transcription was performed using either 3x63RT or 3x66RT (12-mer) primers. PCR was performed by using 3x57R27/3x1F27 (HCV reverse and forward primers, respectively), multiplexed with internal positive control primers (IPCF1 and IPC R1). All RNA genotypes were reverse transcribed with either of the 12-mer RT primers. The results are shown in Table 2 below, with "+" indicating a positive result.

**TABLE 2**

| Sample | Country | Genotype | 3X63RT | 3X66RT | IPC |
|---|---|---|---|---|---|
| BB1-50 | USA | 1 | + | + | + |
| R1156 | Scotland | 2 | + | + | + |
| R1132 | Scotland | 2 | + | + | + |
| T22160 | Tunisia | 3 | + | + | + |
| R1941 | Saudi Arabia | 4 | + | + | + |
| HK505 | Hong Kong | 6 | + | + | + |
| HK791 | Hong Kong | 6 | + | + | + |

Use of 12-mer RT primers, followed by a multiplexed (3' NC and IPC) PCR, resulted in sensitive detection of HCV genotypes. The Roche Monitor® quantitative assay (Roche Diagnostics, Kaiseraugst, Switzerland) detected 50 copies/reaction of genotypes 1 through 6. (Genotype 5 was only tested at 100 copies per reaction. Copy number for this genotype was determined by endpoint dilution.)

### Example 4: Improved Sensitivity Using 13-16 Nucleotide Primers

The following experiment was performed to further optimize detection of HCV using short reverse transcription primers.

RNA from a common HCV positive plasma source was prepared using a modified Purescript® method. RT was performed using one of the following primers were tested: 5'-GTATCAGCACTC-3' <SEQ ID NO.: 2> (designated 66RT12, corresponding to nucleotides 66-77); and extended versions of 66RT12, i.e., 5'-AGTATCAGCACTC-3' <SEQ ID NO.: 3> (66RT13); 5'-CAGTATCAGCACTC-3' <SEQ ID NO. 4> (66RT14); 5'-CCAGTATCAGCACTC-3' <SEQ ID NO. 5> (66RT15); and 5'-GCCAGTATCAGCACTC-3' <SEQ ID NO.: 6> (66RT16).

PCR was performed using 1F27 and 57R27 primers. PCR product was passed over beads that contained either 3x32R25 or 3x30R25 probes. The results are shown in Table 3 below as a percentage of samples yielding positive results.

**TABLE 3**

| RT Length | n=8 50 copies | n=8 3 copies |
|---|---|---|
| 12-mer* | 100% | 66% |
| 13-mer | 100% | 63% |
| 14-mer | 100% | 75% |
| 15-mer | 100% | 88% |
| 16-mer | 100% | 88% |

| | | |
|---|---|---|
| * Results for 12-mer are based on 6 replicates per condition. | | |

This table illustrates the % probe positive results when increasing RT primer lengths are used. Further improvement in copy number sensitivity can be seen when RT primers up to 16 nucleotides in length are used in reverse transcription. Sensitivity differences can be seen when HCV target is used at 3 copies per reaction. As a general observation, greater sensitivity was achieved when using 14 - to 16-mers as the RT primer. Furthermore, there were no significant difference in side-product formation, regardless of copy level or RT primer length. With respect to sensitivity, 3' NC amplification may be more sensitive when reverse transcription is primed with slightly longer RT-primers (e.g. 14 - to 16 - mers). In this experiment, there were no differences in sensitivity at 50 copies of HCV/rxn., but differences were observed at the 3 copy level, with the longer RT primers appearing to provide greater copy number sensitivity.

## Claims

1. A method for reverse transcribing Hepatitis C Virus (HCV) RNA in a biological sample, which method comprises contacting RNA derived from said sample with one or more oligonucleotides under conditions in which said oligonucleotide or oligonucleotides primes synthesis of DNA complementary to at least a portion of said RNA, wherein said oligonucleotide consists of a nucleotide sequence selected from the group consisting of:
(i) SEQ ID NO:1
(ii) SEQ ID NO:2,
(iii) SEQ ID NO:3,
(iv) SEQ ID NO:4,
(v) SEQ ID NO:5,
(vi) SEQ ID NO:6, and
(vii) any combination of the foregoing.

2. A method as defined in claim 1, wherein said sample is selected from the group consisting of blood, serum, plasma, saliva and cerebrospinal fluid.

3. A method as defined in claim 1, further comprising a step of recovering said complementary DNA.

4. A method for detecting the presence of Hepatitis C Virus (HCV) RNA in a biological sample, which method comprises:
(a) performing a reverse transcription reaction according to the method of claim 1;
(b) amplifying products of said reverse transcription reaction to produce amplification products; and
(c) detecting said amplification products;
wherein detection of said amplification products indicates the presence of HCV RNA in said sample.

5. A method as defined in claim 4, wherein said sample is selected from the group consisting of blood, serum, plasma, urine, saliva, and cerebrospinal fluid.

6. A method as defined in claim 4, wherein said amplifying is performed by a method selected from the group consisting of polymerase chain reaction, ligase chain reaction, strand displacement reaction, nucleic acid single base substitution, and transcription mediated amplification.

7. A method as defined in claim 4, wherein said detecting is performed by a method selected from the group consisting of agarose gel electrophoresis, acrylamide gel electrophoresis, colorimetric detection, ECi detection, fluorescence, radioisotopic detection and chemiluminescence.

8. An oligonucleotide consisting of a nucleotide sequence selected from the group consisting of:
(i) SEQ ID NO:1;
(ii) SEQ ID NO:2;
(iii) SEQ ID NO:3;
(iv) SEQ ID NO:4;
(v) SEQ ID NO:5; and
(vi) SEQ ID NO:6.;

9. A kit for detection of HCV in a biological sample, said kit comprising one or more reverse transcription primers according to claim 8.

## Patentansprüche

1. Verfahren zur reversen Transkription von Hepatitis C Virus (HCV)-RNA in einer biologischen Probe, das Verfahren umfassend in Kontakt bringen von RNA, die abgeleitet ist von besagter Probe, mit einem oder mehreren Oligonukleotiden unter Bedingungen, in denen besagtes Oligonukleotid oder Oligonukleotide die Synthese von DNA starten, die komplementär zu mindestens einem Teil der besagten RNA ist, worin besagtes Oligonukleotid aus einer Nukleotidsequenz besteht, die aus der Gruppe ausgewählt ist, bestehend aus:
(i) SEQ ID NO:1
(ii) SEQ ID NO:2,
(iii) SEQ ID NO:3,
(iv) SEQ ID NO:4,
(v) SEQ ID NO:5,
(vi) SEQ ID NO:6 und
(vii) einer Kombination der Vorangegangenen.

2. Verfahren wie in Anspruch 1 definiert, worin besagte Probe ausgewählt ist aus der Gruppe bestehend aus Blut, Serum, Plasma, Speichel und Zerebrospinalflüssigkeit.

3. Verfahren wie in Anspruch 1 definiert, weiterhin umfassend einen Schritt zur Aufreinigung besagter komplementärer DNA.

4. Verfahren zur Detektion der Anwesenheit von Hepatitis C Virus (HCV)-RNA in einer biologischen Probe, wobei das Verfahren umfaßt:
(a) Durchführen einer reversen Transkriptionsreaktion gemäß dem Verfahren von Anspruch 1;
(b) Amplifizieren der Produkte der besagten reversen Transkriptionsreaktion, um Amplifikationsprodukte herzustellen; und
(c) Detektieren besagter Amplifikationsprodukte,
wobei die Detektion besagter Amplifikationsprodukte die Anwesenheit von HCV-RNA in besagter Probe anzeigt.

5. Verfahren wie in Anspruch 4 definiert, worin die besagte Probe ausgewählt ist aus der Gruppe bestehend aus Blut, Serum, Plasma, Urin, Speichel und Zerebrospinalflüssigkeit.

6. Verfahren wie in Anspruch 4 definiert, worin besagtes Amplifizieren mit einer Methode durchgeführt wird, die ausgewählt ist aus der Gruppe bestehend aus Polymerase-Kettenreaktion, Ligase-Kettenreaktion, Strang-Ersetzungsreaktion, Nukleinsäure-Einzelbasen-Substitution und Transkriptions-vermittelte Amplifikation.

7. Verfahren wie in Anspruch 4 definiert, worin das besagte Detektieren mit einer Methode durchgeführt wird, die ausgewählt ist aus der Gruppe bestehend aus Agarose-Gelelektrophorese, Acrylamid-Gelelektrophorese, kolorimetrische Detektion, ECi-Detektion, Fluoreszenz, radioisotopische Detektion und Chemilumineszenz.

8. Oligonukleotid bestehend aus einer Nukleotidsequenz ausgewählt aus der Gruppe bestehend aus:
(i) SEQ ID NO:1
(ii) SEQ ID NO:2,.
(iii) SEQ ID NO:3,
(iv) SEQ ID NO:4,
(v) SEQ ID NO:5 und
(vi) SEQ ID NO:6.

9. Kit zur Detektion von HCV in einer biologischen Probe, besagter Kit umfassend einen oder mehrere reverse Transkriptions-Primer gemäß Anspruch 8.

## Revendications

1. Méthode pour la transcription inverse de l'ARN du virus de l'hépatite C (VHC) dans un échantillon biologique, ladite méthode comprenant la mise en contact d'ARN provenant dudit échantillon avec un ou plusieurs oligonucléotides dans des conditions dans lesquelles ledit ou lesdits oligonucléotides amorcent la synthèse d'ADN complémentaire d'au moins une partie dudit ARN, où ledit oligonucléotide est constitué par une séquence nucléotidique choisie dans le groupe constitué par
(i) SEQ ID n°:1,
(ii) SEQ ID n°:2,
(iii) SEQ ID n°:3,
(iv) SEQ ID n°:4,
(v) SEQ ID n°:5,
(vi) SEQ ID n°:6 et
(vii) une combinaison quelconque des précédents.

2. Méthode selon la revendication 1, dans laquelle ledit échantillon est choisi dans le groupe constitué par le sang, le sérum, le plasma, la salive et le fluide cérébro-spinal.

3. Méthode selon la revendication 1, comprenant en outre une étape de récupération dudit ADN complémentaire.

4. Méthode pour la détection de la présence d'ARN du virus de l'hépatite C (VHC) dans un échantillon biologique, ladite méthode comprenant :
(a) la réalisation d'une réaction de transcription inverse selon la méthode de la revendication 1 ;
(b) l'amplification des produits de ladite réaction de transcription inverse pour produire des produits d'amplification ; et
(c) la détection desdits produits d'amplification,
dans laquelle la détection desdits produits d'amplification indique la présence d'ARN du VHC dans ledit échantillon.

5. Méthode selon la revendication 4, dans laquelle ledit échantillon est choisi dans le groupe constitué par le sang, le sérum, le plasma, l'urine, la salive et le fluide cérébro-spinal.

6. Méthode selon la revendication 4, dans laquelle ladite amplification est réalisée par une méthode choisie dans le groupe constitué par une réaction de polymérisation en chaîne, une réaction de ligature en chaîne par, une réaction de déplacement de brin, une substitution d'une seule base d'acide nucléique et une amplification impliquant une transcription.

7. Méthode selon la revendication 4, dans laquelle ladite détection est réalisée par une méthode choisie dans le groupe constitué par l'électrophorèse sur gel d'agarose, l'électrophorèse sur gel d'acrylamide, la détection colorimétrique, la détection ECi, la fluorescence, la détection radioisotopique et la chimioluminescence.

8. Oligonucléotide constitué par une séquence nucléotidique choisie dans le groupe constitué par
(i) SEQ ID n°:1,
(ii) SEQ ID n°:2,
(iii) SEQ ID n°:3,
(iv) SEQ ID n°:4,
(v) SEQ ID n°:5 et
(vi) SEQ ID n°:6.

9. Kit de détection du VHC dans un échantillon biologique, ledit kit comprenant une ou plusieurs amorces de transcription inverse selon la revendication 8.
